# EUROPEAN PATENT APPLICATION

(11) **EP 2 966 446 A1**
(43) Date of publication of application: **13.01.2016**
(21) Application number: 14861441.5
(22) Date of filing: 07.03.2014
(51) Int. Cl.: G01N 33/48, G06F 19/00

(54) **IDENTIFICATION CALIBRATION METHOD FOR BLOOD GAS BIOCHEMICAL ANALYSIS SYSTEM, AND DEVICE**

(30) Priority: 24.01.2014 CN 201410038097
(71) Applicant: Edan Instruments, Inc., Nanshan, Shenzhen 518067 (CN)
(72) Inventor: XIE, Yingying, Shenzhen Guangdong 518067 (CN); LIN, Chao, Shenzhen Guangdong 518067 (CN); ZHAO, Zhixiang, Shenzhen Guangdong 518067 (CN)
(74) Representative: ProI European Patent Attorneys
(86) International application number: PCT/CN2014/073053
(87) International publication number: WO 2015/070548

(57) **Abstract**

The present invention relates to a recognition and calibration method for the blood gas chemistry analytic system, which comprises the following steps: getting reagent information recorded in the two-dimension code glued to or printed on the test card or reagent package; extracting the basic information of the test card from the reagent information, examining the correctness of the usage of the test card and feeding back the error messages and exiting the test if the usage is incorrect; if the usage is correct, extracting the feature information of the test card from the reagent information, obtaining the feature parameters of the test card and setting them as the feature parameters of the present test; collecting the present test signals and processing the operation of computation and adjustment for the signals according to the feature parameters of the present test and thus, obtaining the test results.

## Description

### FIELD OF THE INVENTION

The present invention relates to the test apparatus and relevant reagent for medical purpose. More particularly, it relates to a recognition and calibration method for the blood gas chemistry analytic system and an apparatus implementing the method.

### BACKGROUND OF THE INVENTION

The blood gas chemistry analytic system usually includes blood gas chemistry analyzer and supporting reagent (e.g. test card, reagent package, etc.). The blood gas chemistry analyzer obtains signals generated from the reaction between the extracorporeal sample of human or animal body (e.g. blood, plasma, serum, urine, interstitial fluid, etc.) and the reagent and it provides references to medical care by giving the information of analyte of the sample, such as concentration. The premise that the gained information of analyte of the sample (e.g. concentration) is accurate and reliable is that the blood gas chemistry analyzer obtains the feature information (e.g. calibration target value, standard curve, calibration parameter, etc.) of the tested reagent comprehensively and accurately. So far, in the market, the programs of most of the blood gas chemistry analytic systems are written in default reagent information (e.g. calibration target value, standard curve, calibration parameter, etc.) which corresponds with the attribute values of analyte of the sample (e.g. concentration). That requires the features of reagent from every batch must be highly consistent, which calls for extremely strict techniques and higher cost for producing reagent. Besides, it is very difficult to keep reagent features of all the batches the same in practical productive process and once a slight change occurs in any of the coming batch, the apparatus software have to be upgraded so that the test accuracy can be guaranteed. That makes it harder for users to use and maintain the apparatus. Thus, the test cost of this type of system is very high and the accuracy of the test results cannot be guaranteed.

### SUMMARY OF THE INVENTION

The present invention is directed to technical defects of the prior art of "high cost for testing and difficulty to guarantee accuracy" and to provide a recognition and calibration method for the blood gas chemistry analytic system and the apparatus.

The technical scheme adopted in the present invention is to establish a recognition and calibration method for the blood gas chemistry analytic system, comprising the following steps:

A) getting the reagent information recorded in the two-dimension code glued to or printed on the test card or reagent package;

B) extracting the basic information of the test card from the reagent information, examining the correctness of the usage of the test card; if the usage is correct, proceeding to the next step; if the usage is incorrect, feeding back the error messages and exiting the test;

C) extracting the feature information of the test card from the reagent information, obtaining the feature parameters of the test card and setting a feature parameter function of the present test;

D) collecting the present test signals, putting the signals into the feature parameter function and processing the operation of computation and thus, obtaining the test results.

Further, said step B) further comprising:

B1) extracting the type information of the test card from the reagent information and examining if the type information matches the present test type; if not matching, feeding back the error messages and exiting the test; if matching, proceeding to the next step;

B2) extracting the information of valid period of the test card from the reagent information and examining if the present period is within the validity; if not, feeding back the error messages and exiting the test; if within, proceeding to the next step;

wherein, the said present test type is the test type used in the present system via test card; and the said present time is the actual time that the system is in at present.

Further, said step C) further comprising:
C1) obtaining the standard curve parameters denoting the corresponding relationship between the collected responding signals and the test results, and the calibration parameters denoting the corresponding relationship between the test environment parameters and the test results, from the test card;
C2) generating the standard curve function and the calibration parameter function of the test card by using the standard curve parameters and the calibration parameters respectively.

Further, the standard curve function and the calibration parameter function of step C2) are polynomials that respectively take the test results and the calibration results as the dependent variables, the sample signals and the test results as independent variables, and the standard curve parameters and the calibration parameters as coefficients.

Further, said step D) further comprising:
D1) obtaining the calibration responding signals and the sample responding signals;
D2) putting the calibration responding signals and the sample responding signals into the standard curve function and getting the test results of the present test;
D3) substituting the test results into the calibration parameter function, to get the calibration results.

Further, the reagent information in step A) also contains the information of the reagent package; before conducting step D), the following steps are conducted:
M) extracting the basic information of the reagent package from the reagent information, examining the correctness of the usage of the reagent package; if the usage is correct, proceeding to the next step; if the usage is incorrect, feeding back the error messages and exiting the test;
N) extracting the feature information of the reagent package from the reagent information, obtaining the feature parameters of the reagent package and setting them as the feature parameters of the present test; Parameters of the reagent package include calibration target values and calibration parameters.

Further, the said step D) also includes the following steps:
D0) employing the calibration target value and calibration signals for calibration.

The present invention also relates to an apparatus implementing the recognition and calibration method for the blood gas chemistry analytic system, comprising the following:
Retrieval module for the reagent information: used for getting the reagent information recorded in the two-dimension code glued to or printed on the test card or reagent package;
Examination module for the basic information of the test card: used for extracting the basic information of the test card from the reagent information, examining the correctness of the usage of the test card; if the usage is correct, proceeding to the analysis module for the feature information of the test card; if the usage is incorrect, feeding back the error messages and exiting the test;
Analysis module for the feature information of the test card: used for extracting the feature information of the test card from the reagent information, obtaining the feature parameters of the test card and setting them as the feature parameters of the present test;
Data collection and processing module: used for collecting the present test signals and processing the operation of computation and adjustment for the signals according to the feature parameters of the present test and thus, obtaining the test results.

Further comprising:
Examination module for the basic information of the reagent package: used for extracting the basic information of the reagent package from the reagent information, examining the correctness of the usage of the reagent package; if the usage is correct, proceeding to the analysis module for the feature information of the reagent package; if the usage is incorrect, feeding back the error messages and exiting the test;
Analysis module for the feature information of the reagent package: used for extracting the feature information of the reagent package from the reagent information, obtaining the feature parameters of the reagent package and setting them as the feature parameters of the present test; the parameters of the said reagent package include calibration target values and calibration parameters.

Further, the examination module for the basic information of the test card and the analysis module for the feature information of the test card form the information module of the test card, or, the examination module for the basic information of the reagent package and the analysis module for the feature information of the reagent package form the information module of the reagent package.

Employing the recognition and calibration method for the blood gas chemistry analytic system and the apparatus of this invention has the following benefits: Because the product type, batch number and period of validity can be obtained via the reagent information, it can be examined automatically that if the usage of the product is correct or if the product is in the period of validity for various reagent products. Thus, incorrect usage executed by users can be avoided and usability of the product can be improved. Meanwhile, the calibration target value, standard curve and calibration parameter, etc. can be obtained via the reagent information and for different reagent products, the system calibration, result computation and result calibration can be executed automatically so that the precision and accuracy of the test results can be improved. Moreover, the requirement of consistency between reagent product batches is no longer that high, which means the requirement of producing techniques for the reagent product is lowered. In this way, the producing cost of reagent product is reduced and the producing efficiency of reagent product is improved. Therefore, the test cost is lower and the results are more accurate.

### BRIEF DESCRIPTION OF THE DRAWINGS

Fig. 1 is the implementing flow chart of an embodiment of the recognition and calibration method for the blood gas chemistry analytic system and the apparatus implementing the method in the invention;
Fig. 2 is the method flow chart of the embodiment under some circumstances;
Fig. 3 is the structure diagram of an apparatus of the embodiment.

### DETAILED DESCRIPTION OF THE PREFERRED EMBODIMENTS

The present invention will be further described in details in combination with the drawings and specific embodiments.

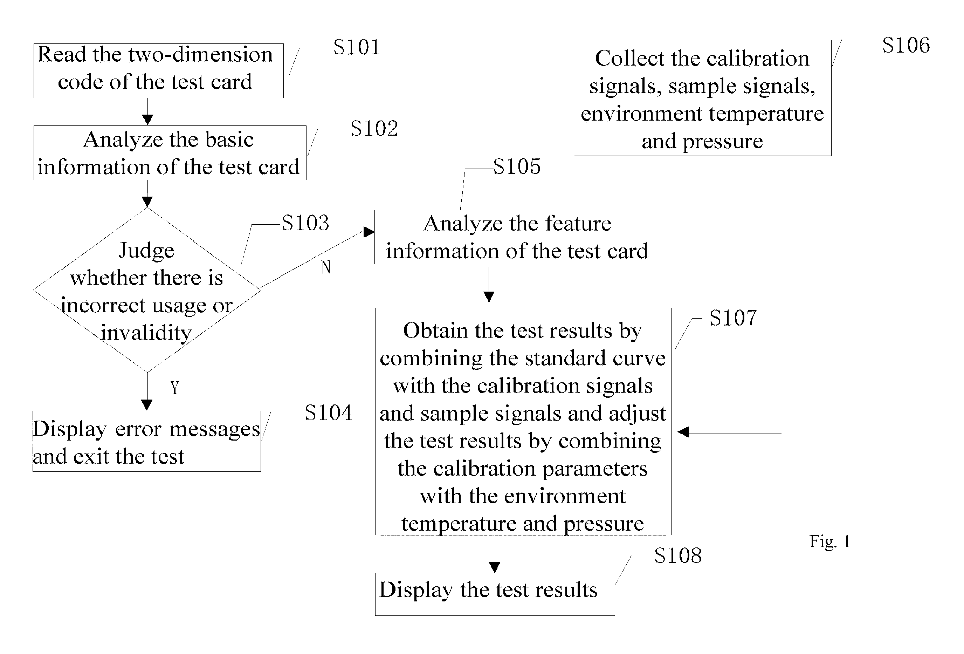

As shown in Fig. 1, the embodiment of the recognition and calibration method for the blood gas chemistry analytic system and the apparatus implementing the method in the invention includes the following steps:
Step S101 Read the two-dimension code of the test card: in this step, reagent information recorded in the carrier is obtained. In this embodiment, the reagent information is obtained by scanning the two-dimension code of the test card and the reagent package, which means the information of the test card is read via scanning the two-dimension code on the test card or on the package of the test card. The said two-dimension code may include not just the information of the test card, but in this step, only the information of the test card is extracted.
Step S102 Analyze the basic information of the test card: in this step, the information of the test card is analyzed and the basic information of the test card is extracted. In this embodiment, the basic information of the test card includes product type, batch number, period of validity, etc.
Step S103 Judge whether there is incorrect usage or invalidity: if there is, proceeding to S104; if there is not, proceeding to S105.

The judging process of this step can be further described: extracting the type information of the test card from the reagent information and examining if it matches the present test type, if not, proceeding to S104 and feeding back the error messages and exiting the test; if matching, extracting the validity information of the test card from the reagent information and examining if the present period is in validity, if not, proceeding to S104 and feeding back the error messages and exiting the test, if the present period is in validity, proceeding to S105; the present test type is the test type employed by the present system via test card; the present time is the actual time that the system is in at present.
Step S104 Display error messages and exit the test: in this step, if the usage of the test card is incorrect or invalid, the error messages will be sent to the users and the test will be terminated.
Step S105 Analyze the feature information of the test card: in this step, as examination has been made in Step S103 and it is assured that the usage of the test card is correct and valid, further analysis can be conducted on the gained information of the test card and the feature information of the test card can be obtained. In this embodiment, the feature information of the test card includes standard curve, calibration parameter, etc. The standard curve of the test card is obtained by conducting sampling experiment with statistical significance towards the test cards in the same batch, testing the known standard quality control samples, recognizing the specific relationship between the responding signals (e.g. calibration signal, sample signal) of the test cards in this batch and the test results (e.g. concentration of the sample analyte), which means the corresponding relationship between the responding signals and the test results. The calibration parameters of the test card are obtained by conducting sampling experiment with statistical significance under various temperatures and pressures, testing the known standard quality control samples and recognizing the specific relationship between the test results (e.g. concentration of the sample analyte) and the environment temperatures and pressures, which means the deviation of test results occurring accompanied by various test environment parameters. In this embodiment, the standard curve is used for adjusting the curves when the features of the test cards change and thus the obtained feature information of the test card is more accurate and the test results (e.g. concentration of the sample analyte) are more accurate. Moreover, the requirement of consistency between batches is lower and the requirement of producing techniques for the test cards is also lower, and the producing cost is lower. The calibration parameters are used for adjusting the test results (e.g. concentration of the sample analyte) according to the actual environment temperature and pressure in order to make the test results (e.g. concentration of the sample analyte) more accurate.

In this embodiment, this step can be further described: obtaining the standard curve parameters denoting the corresponding relationship between the collected responding signals and the test results, and the calibration parameters denoting the corresponding relationship between the test environment parameters and the test results, from the test card; generating the standard curve function and the calibration parameters function of the test card by using the standard curve parameters and the calibration parameters respectively. For example, the equation generating the test result y0 is y0=ax2+bx+c (an example and there are more), wherein (a, b, c) are the standard curve parameters extracted from the test card feature information in the two-dimension code and (x) is the collected responding signals, which means that y0=f(x, a,b,c) is that standard curve parameter function. Substitute the standard curve parameters (a, b, c; obtained from the said two-dimension code) and the responding signals (x; obtained from the system sample) into the above function once the parameters and signals are obtained, to get the test result y0.

Besides, in the following steps, the adjusted result (y) is expressed as y= y0+eT+fP (an example and there are more), wherein the (e, f) are calibration parameters extracted from the test card feature information in the two-dimension code and (T) is the collected environment temperature and (P) is the pressure, which means that y=f(y0, T,P,e,f) is that calibration parameter function. Substitute the calibration parameters (e, f), the environment temperature (T) and the pressure (P) into the above function once the data are obtained, to adjust y0 to y. The way dealing with the feature information of the reagent package (g, h, i) and the calibration signals (x0) and the environment temperature (T) and the pressure (P) is similar to the way said above.
Step S106 Collect the calibration signals, sample signals, environment temperature and pressure: in this embodiment, this step is the actual test step which tests the sample via test card and obtain relevant test data, To be particular, it is the sensor on the test card that senses the calibration responding signals and the sample signals and it is the sensor in the apparatus that senses the environment temperature and pressure and the signal collecting module in the apparatus collects these signals.
Step S107 Obtain the test results by combining the standard curve with the calibration signals and sample signals and adjust the test results by combining the calibration parameters with the environment temperature and pressure: in this step, employing the information (or data) of the standard curve and combining it with the calibration responding signals and sample responding signals obtained in S106 lead to the test results (e.g. concentration of the sample analyte) of the present sample. On this basis, employing the said calibration parameters and combining them with the present environment temperature and pressure lead to adjustment of the present test results (e.g. concentration of the sample analyte). Therefore, with the accurate computation and precise adjustment, accurate and reliable test results (e.g. concentration of the sample analyte) of the present sample can be obtained. More particularly, this step can be further described: obtaining the calibration responding signals and sample responding signals in the test; substituting the calibration responding signals and sample responding signals into the said standard curve parameter function and obtaining the test results of the present test; substitute the present environment parameters and the test results into the calibration parameter function to adjust the test results and gain the adjusted test results.
Step S108 Display the test results: in this step, the final accurate and reliable test results (e.g. concentration of the sample analyte) of the present sample are displayed.

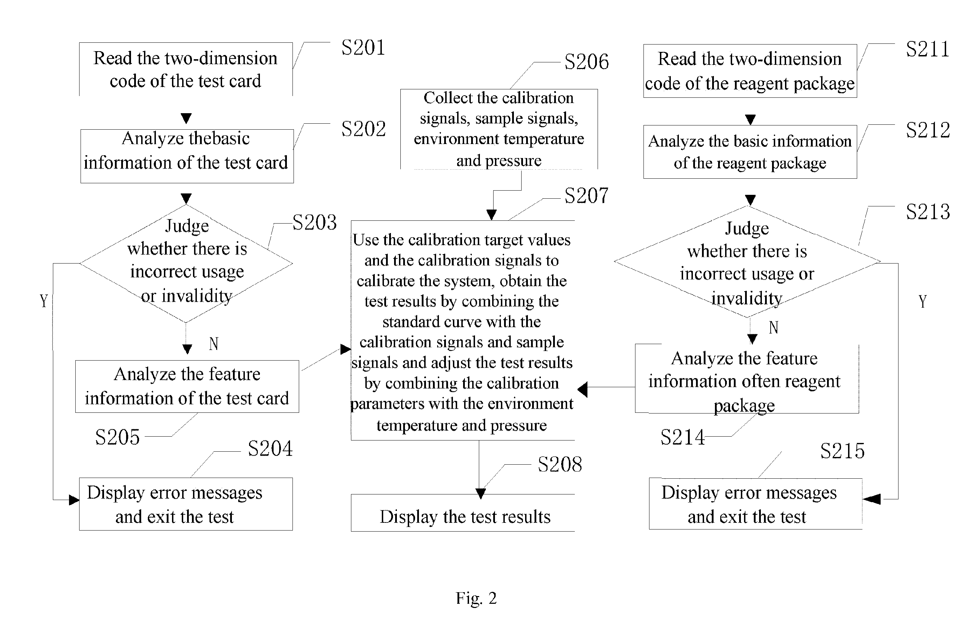

Under some circumstances in this embodiment, the technological process of this method can be slightly different with the said one and for understanding that, please refer to Fig. 2. Fig. 2 contains Step S201-Step S215, wherein the Steps S201-S208 are the same as the Step S101-S108 (except that there is a slight difference between S207 and S107), the Step S211-S215 need to be conducted before S207 and there is a slight difference between S207 and S107. Therefore, the contents of Steps S201-S206 and Step S208 are omitted (refer to the descriptions above). And the contents of Step S211-S215 will be described in detail, as below:
Step S211 Read the two-dimension code of the reagent package: in this step, the two-dimension code on the reagent package or the casting of the reagent package is scanned and the information of the reagent package is read. In this embodiment, reading the two-dimension code of the reagent package in this step can be conducted both at the same time and not at the same time as reading the two-dimension code of the test card in Step S201. It only needs to be conducted before Step S207. Essentially speaking, Step S211 does also work for extracting the reagent information recorded in the carrier.
Step S212 Analyze the basic information of the reagent package: in this step, the information of the reagent package is analyzed and the basic information of the reagent package is extracted. In this embodiment, the basic information of the reagent package includes product type, batch number, period of validity, etc.
Step S213 Judge whether there is incorrect usage or invalidity: if there is, proceeding to S214; if there is not, proceeding to S215. The judgment of incorrect usage and invalidity is made according to the basic information of the reagent package such as product type, batch number, period of validity, etc.
Step S214 Display error messages and exit the test: in this step, if the usage of the reagent package is incorrect or invalid, the error messages will be sent to the users.
Step S215 Analyze the feature information of the reagent package: in this step, as examination has been undertaken and it is assured that the usage of the reagent package is correct and valid, further analysis can be conducted on the information of the reagent package obtained in Step S211 to obtain the feature information of the reagent package. In this embodiment, the feature information of the reagent package includes calibration target values, calibration parameters, etc. The calibration target values are obtained by conducting sampling experiment with statistical significance towards the reagent packages in the same batch and recognizing specific component content in this batch. In this way, the calibration target values can be adjusted according to the deviations of features between batches and the obtained feature information of reagent package can be more accurate. The test results (e.g. concentration of the sample analyte) are more accurate. The requirement of consistency between batches is lower and the requirement of producing techniques for the test cards is also lower. And the producing cost is lower. The calibration parameters of the reagent package are obtained by conducting sampling experiment with statistical significance under various temperatures and pressures, and confirming their effects on the test results (e.g. concentration of the sample analyte) under various temperatures and pressures. In this case, the test results (e.g. concentration of the sample analyte) can be adjusted according to the actual environment temperature, pressure, etc. and they can be more accurate.

In Fig. 2, the Step S207 has some extra content compared with the Step S107, which is using the calibration target values and the calibration signals to calibrate the system. That is, in Step S207, the obtained calibration target values are firstly matched with the present calibration signals to calibrate the present system. For example, if a calibration target value revealing the concentration of the analyte in the calibration solution of the reagent package - C0 corresponds with the present calibration signal voltage - 5mV or the present calibration signal current - -100nA, a direct and accurate mapping relation should be established between the present calibration signal and the concentration of the analyte in the calibration solution - C0, in order to calibrate the present system. Except the above operation for calibrating the system, the other operations in Step S207 are the same as those in Step S107.

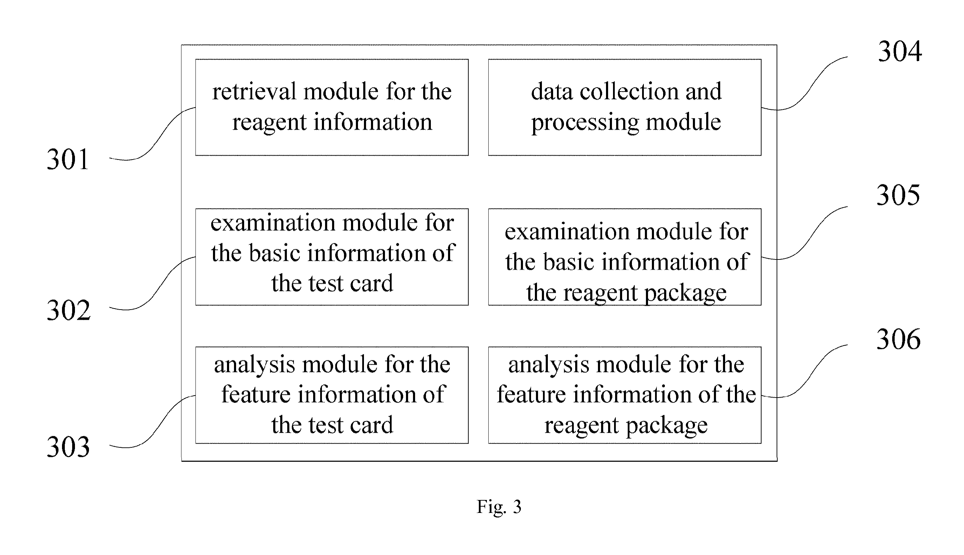

This embodiment also relates to an apparatus implementing the above method, the structure of which is displayed in Fig. 3. In Fig. 3, this apparatus includes: retrieval module for the reagent information 301, examination module for the basic information of the test card 302, analysis module for the feature information of the test card 303, data collection and processing module 304, examination module for the basic information of the reagent package 305 and analysis module for the feature information of the reagent package 306. Wherein, the retrieval module for the reagent information 301 is used for getting the reagent information recorded in the carriers; examination module for the basic information of the test card 302 is used for extracting the basic information of the test card from the reagent information, examining the correctness of the usage of the test card and if the usage is correct, proceeding to the analysis module for the feature information of the test card 303 and if the usage is incorrect, feeding back the error messages and exiting the test; analysis module for the feature information of the test card 303 is used for extracting the feature information of the test card from the reagent information, obtaining the feature parameters of the test card and setting them as the feature parameters of the present test and the said feature parameters include standard curve and calibration parameters, etc.; data collection and processing module 304 is used for collecting the present test signals and processing the operation of computation and adjustment for the signals according to the feature parameters of the present test and thus, obtaining the test results. Examination module for the basic information of the reagent package 305 is used for extracting the basic information of the reagent package from the reagent information, examining the correctness of the usage of the reagent package and if the usage is correct, proceeding to the analysis module for the feature information of the reagent package and if the usage is incorrect, feeding back the error messages and exiting the test; analysis module for the feature information of the reagent package 306 is used for extracting the feature information of the reagent package from the reagent information, obtaining the feature parameters of the reagent package and setting them as the feature parameters of the present test and the parameters of the reagent package include calibration target values and calibration parameters. In this embodiment, the retrieval module for the reagent information 301 obtains the reagent information by scanning the information recorded in the two-dimension code glued to or printed on the test card or reagent package;

In addition, it is worth mentioning that, in this embodiment, the examination module for the basic information of the reagent package 305 and the analysis module for the feature information of the reagent package 306 are optional. That is, in this embodiment, it is reasonable that under some circumstances, there are just the said retrieval module for the reagent information 301, examination module for the basic information of the test card 302, analysis module for the feature information of the test card 303 and data collection and processing module 304.

Besides, under some circumstances, the examination module for the basic information of the test card 302 and the analysis module for the feature information of the test card 303 can merge into one, which means a test card information module is built to substitute the above two. Similarly, the examination module for the basic information of the reagent package 305 and the analysis module for the feature information of the reagent package 306 can merge into one and a reagent package information module is built (not shown in the figures).

The above are only preferred embodiments of the present invention. It should be noted that for those of ordinary skill in the art, under the premise without departing from the principle of the invention, further improvements and modifications may be made. All these improvements and modifications should also be regarded within the protection scope of the present invention.

## Claims

1. A recognition and calibration method for the blood gas chemistry analytic system, comprising the following steps of:
A) getting the reagent information recorded in the two-dimension code glued to or printed on the test card or reagent package;
B) extracting the basic information of the test card from the reagent information, examining the correctness of the usage of the test card; if the usage is correct, proceeding to the next step; if the usage is incorrect, feeding back the error messages and exiting the test;
C) Extracting the feature information of the test card from the reagent information, obtaining the feature parameters of the test card and setting a feature parameter function of the present test; and
D) Collecting the present test signals, putting the signals into the feature parameter function and processing the operation of computation and thus, obtaining the test results.

2. The recognition and calibration method for the blood gas chemistry analytic system according to claim 1, wherein step B) further comprises the following steps of:
B1) extracting the type information of the test card from the reagent information and examining if the type information matches the present test type; if not matching, feeding back the error messages and exiting the test; if matching, proceeding to the next step;
B2) extracting the information of valid period of the test card from the reagent information and examining if the present period is within the validity; if not, feeding back the error messages and exiting the test; if within, proceeding to the next step; wherein, the said present test type is the test type used in the present system via test card; and the said present time is the actual time that the system is in at present.

3. The recognition and calibration method for the blood gas chemistry analytic system according to claim 1, wherein step C) further comprises the following steps of:
C1) obtaining the standard curve parameters denoting the corresponding relationship between the collected responding signals and the test results, and the calibration parameters denoting the corresponding relationship between the test environment parameters and the test results, from the test card; and
C2) generating the standard curve function and the calibration parameter function of the test card by using the standard curve parameters and the calibration parameters respectively.

4. The recognition and calibration method for the blood gas chemistry analytic system according to claim 3, wherein the standard curve function and the calibration parameter function of step C2) are polynomials that respectively take the test results and the calibration results as the dependent variables, the sample signals and the test results as independent variables, and the standard curve parameters and the calibration parameters as coefficients.

5. The recognition and calibration method for the blood gas chemistry analytic system according to claim 1, wherein step D) further comprises the following steps of:
D1) obtaining the calibration responding signals and the sample responding signals;
D2) putting the calibration responding signals and the sample responding signals into the standard curve function and getting the test results of the present test; and
D3) substituting the test results into the calibration parameter function, to get the calibration results.

6. The recognition and calibration method for the blood gas chemistry analytic system according to claim 1, wherein the reagent information in step A) also contains the information of the reagent package; before conducting step D), the following steps are conducted:
M) extracting the basic information of the reagent package from the reagent information, examining the correctness of the usage of the reagent package; if the usage is correct, proceeding to the next step; if the usage is incorrect, feeding back the error messages and exiting the test; and
N) extracting the feature information of the reagent package from the reagent information, obtaining the feature parameters of the reagent package and setting them as the feature parameters of the present test; parameters of the reagent package include calibration target values and calibration parameters.

7. The recognition and calibration method for the blood gas chemistry analytic system according to claim 1, wherein step D) also includes the following step of:
D0) employing the calibration target value and calibration signals for calibration.

8. An apparatus implementing the recognition and calibration method for the blood gas chemistry analytic system as claimed in claim 1, comprising the following:
retrieval module for the reagent information: used for getting the reagent information recorded in the two-dimension code glued to or printed on the test card or reagent package;
examination module for the basic information of the test card: used for extracting the basic information of the test card from the reagent information, examining the correctness of the usage of the test card; if the usage is correct, proceeding to the analysis module for the feature information of the test card; if the usage is incorrect, feeding back the error messages and exiting the test;
analysis module for the feature information of the test card: used for extracting the feature information of the test card from the reagent information, obtaining the feature parameters of the test card and setting them as the feature parameters of the present test; and
data collection and processing module: used for collecting the present test signals and processing the operation of computation and adjustment for the signals according to the feature parameters of the present test and thus, obtaining the test results.

9. The apparatus according to claim 8, wherein the followings are also included:
examination module for the basic information of the reagent package: used for extracting the basic information of the reagent package from the reagent information, examining the correctness of the usage of the reagent package; if the usage is correct, proceeding to the analysis module for the feature information of the reagent package; if the usage is incorrect, feeding back the error messages and exiting the test; and
analysis module for the feature information of the reagent package: used for extracting the feature information of the reagent package from the reagent information, obtaining the feature parameters of the reagent package and setting them as the feature parameters of the present test; the parameters of the said reagent package include calibration target values and calibration parameters.

10. The apparatus according to claim 9, wherein the examination module for the basic information of the test card and the analysis module for the feature information of the test card form the information module of the test card, or, the examination module for the basic information of the reagent package and the analysis module for the feature information of the reagent package form the information module of the reagent package.
